# EUROPEAN PATENT APPLICATION

(11) **EP 3 195 818 A1**
(43) Date of publication of application: **26.07.2017**
(21) Application number: 16168215.8
(22) Date of filing: 13.08.2009
(51) Int. Cl.: A61B 17/221

(54) **APPARATUS AND METHODS FOR RETRIEVING AN OBJECT FROM A BODY PASSAGE**

(30) Priority: 14.08.2008 US 88848 P
(62) Divisional of application: 09748555.1
(71) Applicant: United States Endoscopy Group, Inc., Mentor, OH 44060 (US)
(72) Inventor: MERRIFIELD, Benjamin F., OLYMPIA, WA Washington 98502 (US)
(74) Representative: Theander, Anna Katarina Henrietta

(57) **Abstract**

The present invention provides apparatus and methods for retrieving an object from a body passage. In one embodiment, the apparatus comprises a first tube member, and optionally a second tube member. A proximal region of an arm is secured in place, while a distal region of the arm is coupled to a loop member of a snare. In use, proximal and distal advancement of a control member coupled to the loop member moves the loop member between collapsed and expanded states, respectively. In the collapsed state, the arm preferably does not extend radially beyond an outer diameter of the first tube member, thereby facilitating advancement of the retrieval device to a target site, e.g., via an endoscope. In the expanded state, the arm may move away from the first and second tube members, facilitate positioning of the loop member, and may facilitate capture of the object from the body passage.

## Description

### PRIORITY CLAIM

This invention claims the benefit of priority of U.S. Provisional Application Serial No. 61/088,848, entitled "Foreign Body Retrieval Device," filed August 14, 2008, the disclosure of which is hereby incorporated by reference in its entirety.

### BACKGROUND

There are many instances in which it may be desirable or necessary to remove an object from a passage of a patient, including without limitation the esophagus, stomach, colon, lungs, or common bile duct. Different objects may be present from different conditions and in different passages.

As one example, various calculi, or "stones," may form within body passages, such as kidney stones in the ureter or kidneys, and gallstones in bile ducts or the gallbladder. Some stones may be harmless and may pass through the body naturally, for example, gallstones passing through the duodenum and kidney stones through the urethra. However, many other stones may become trapped and may cause serious medical problems, such as abdominal pain, fever, nausea, jaundice, and so forth. Fast and effective removal of such stones may become necessary.

In another example, it may become necessary or desirable to remove colorectal polyps. A colonoscopy or sigmoidoscopy may be performed to detect the existence of polyps. A doctor is likely to remove all polyps discovered during a bowel examination, and once removed, a pathologist may examine the polyps under a microscope to determine whether the polyps are cancerous. One present technique for removing polyps is to use a looped snare. In this technique, a wire loop cuts the base of the polyp and cauterizes it to prevent bleeding. This requires looping the snare over the head of the polyp, then positioning the loop over the base prior to cauterization. After the polyp has been cut, a retrieval device, such as a forceps, may be used to capture and remove the polyp.

The foregoing are merely two examples of various instances in which it may be necessary or desirable to remove an object from a body passage. In each instance, it is important to secure the object during removal, regardless of its size or characteristics, to reduce or eliminate the possibility of the object escaping during withdrawal from the passage.

### SUMMARY

The present invention provides apparatus and methods for retrieving an object from a body passage. In one embodiment, the apparatus comprises first tube member having proximal and distal regions and a lumen extending therebetween, and optionally, a second tube member extending distally from the first tube member. The apparatus further comprises an arm having proximal and distal regions. The proximal region of the arm may be secured in place, while the distal region of the arm is coupled to a loop member of a snare, which in turn is coupled to a control member.

In use, proximal and distal advancement of a control member coupled to the loop member moves the loop member between collapsed and expanded states, respectively. In the collapsed state, the arm preferably does not extend radially beyond an outer diameter of the first tube member, thereby facilitating advancement of the retrieval device to a target site, e.g., via an endoscope. In the expanded state, the arm may move away from the first and second tube members, facilitate positioning of the loop member, and may facilitate capture of the object from the body passage.

The arm may comprise a substantially rectangular cross-sectional profile, or alternatively, a concave curvature. In one embodiment, a width of the arm is about 0.2 to about 0.95 times an outer diameter of either the first or the second tube member. The provision of an arm that is relatively wide may facilitate capture and removal of the object from the body passage.

In various alternative embodiments, the first tube member or the second tube member may comprise a groove that is sized to receive the arm in the collapsed state, such that the arm is substantially flush with the device and does not increase its delivery profile. Alternatively, the arm may be formed integrally with the first tube member or the second tube member by providing two longitudinal slits in the distal end of either tube member. In yet a further alternative, first and second arms may be coupled to the loop member at multiple locations.

Other systems, methods, features and advantages of the invention will be, or will become, apparent to one with skill in the art upon examination of the following figures and detailed description. It is intended that all such additional systems, methods, features and advantages be within the scope of the invention, and be encompassed by the following claims.

### BRIEF DESCRIPTION OF THE DRAWINGS

The invention can be better understood with reference to the following drawings and description. The components in the figures are not necessarily to scale, emphasis instead being placed upon illustrating the principles of the invention. Moreover, in the figures, like referenced numerals designate corresponding parts throughout the different views.
FIGS. 1A-1B are side views of a first embodiment of a retrieval device in collapsed and expanded states, respectively.
FIGS. 2A-2B are side-sectional views of a distal region of the retrieval device of

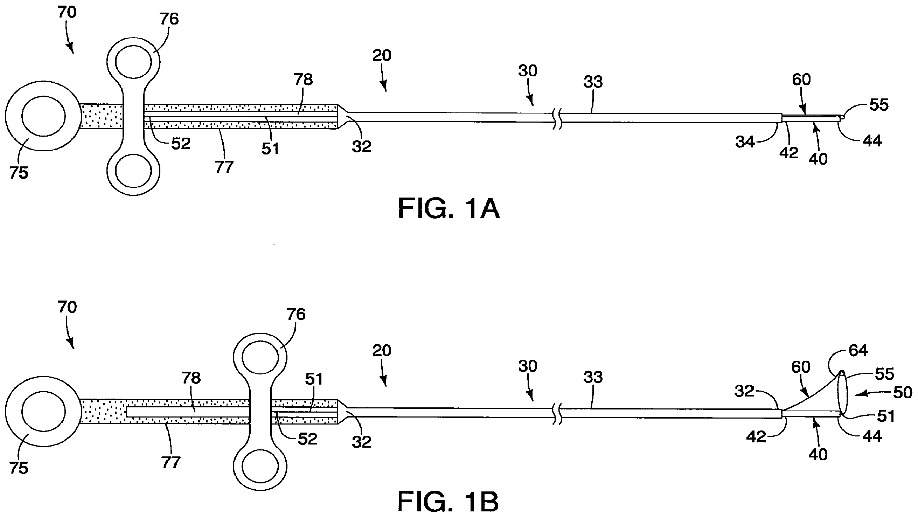

FIGS. 1A-1B in collapsed and expanded states, respectively.

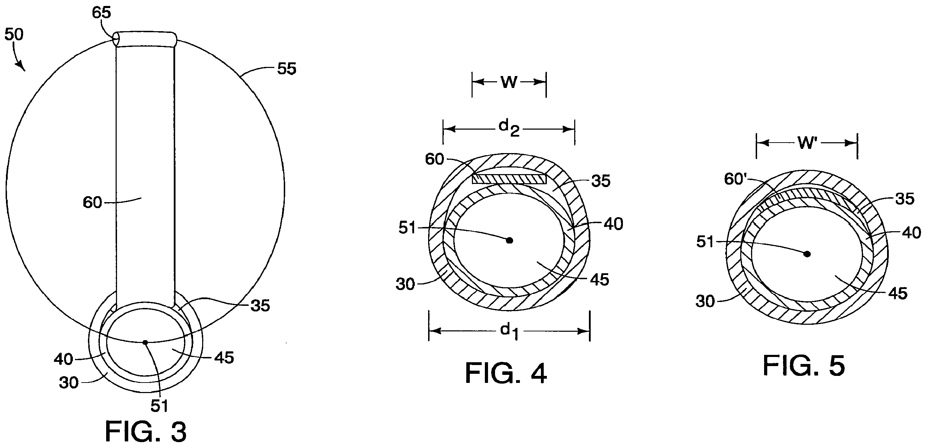

FIG. 3 is an end view of the retrieval device of FIGS. 1-2 in an expanded state.

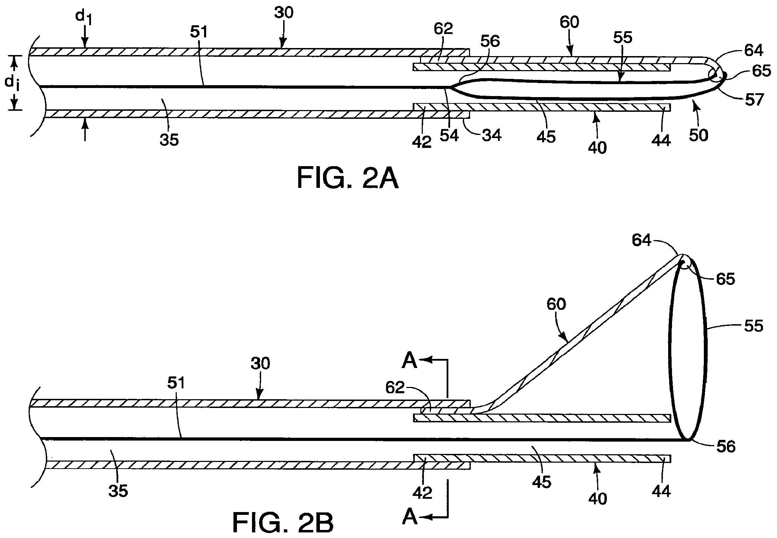

FIG. 4 is a first cross-sectional view along line A-A of FIG. 2B.
FIG. 5 is an alternative cross-sectional view along line A-A of FIG. 2B.

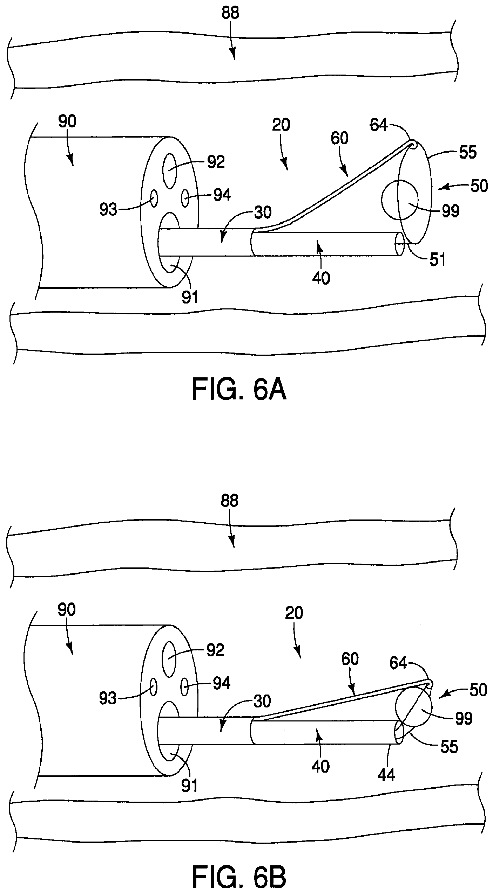

FIGS. 6A-6B are perspective views of an exemplary method of capturing an object using the retrieval device of FIGS. 1-5.

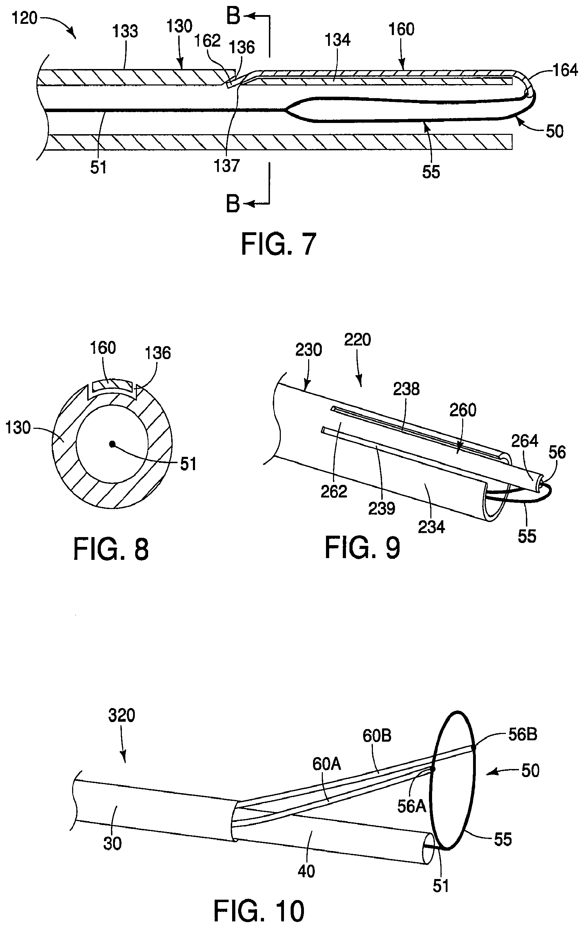

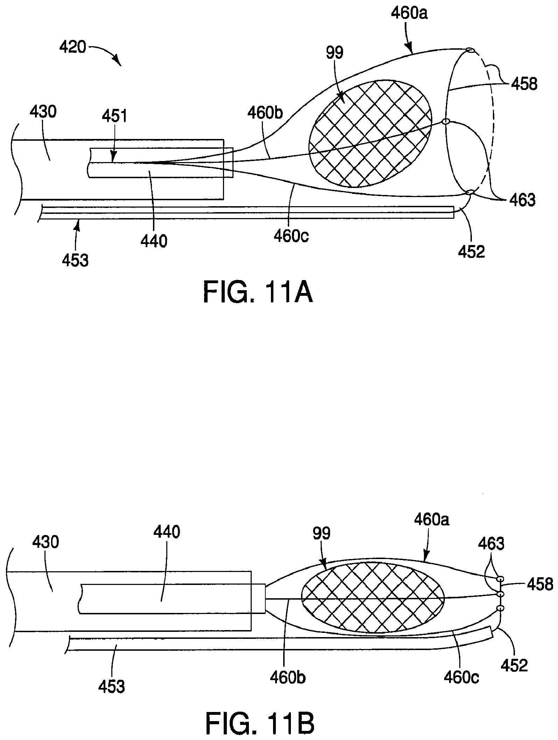

FIG. 7 is a side-sectional view of a distal region of an alternative retrieval device in a collapsed state.
FIG. 8 is a cross-sectional view along line B--B of FIG. 7.
FIG. 9 is a perspective view of an alternative retrieval device in a collapsed state.
FIG. 10 is a perspective view of a further alternative retrieval device in an expanded state.
FIGS. 1 IA-I IB are schematic side views of a further alternative retrieval device in open and closed states, respectively.

### DETAILED DESCRIPTION OF THE PREFERRED EMBODIMENTS

In the present application, the term "proximal" refers to a direction that is generally towards a physician during a medical procedure, while the term "distal" refers to a direction that is generally towards a target site within a patent's anatomy during a medical procedure.

Referring to FIGS. 1-6, a first embodiment of a retrieval device 20 is shown. In this embodiment, the retrieval device 20 comprises a first tube member 30, a second tube member 40, a snare 50 and an arm 60. However, as will be explained further in alternative embodiments below, the second tube member 40 may optionally be omitted.

As will be explained in greater detail below, the arm 60 of the retrieval device 20 provides several important clinical advantages. The arm 60 is different than a thread-like element by comprising an increased width (shown as w or w' below) that significantly enhances grasping and pinching of objects to facilitate secure removal of the objects. The arm 60 also may be inclined to deploy to a predetermined or biased shape to promote a consistent expanded configuration of the loop member 55. Further, the arm 60 may have an enhanced column strength that may allow the retrieval device 20 to be driven and/or pushed beyond objects in a passage when in an expanded state, and may reduce the risk of collapse of the arm 60 and snare 50 in the expanded state. Thus, there is a significantly enhanced pushability of the snare 50 and the arm 60 in the expanded state relative to a thread-like element coupled to the snare.

The first tube member 30 has proximal and distal regions 32 and 34, respectively, and a central region 33 therebetween. Further, a lumen 35 extends between the proximal and distal regions 32 and 34. The first tube member 30 may comprises a relatively flexible, tubular member that may be formed from one or more semi-rigid polymers. For example, the first tube member 30 may be manufactured from polyurethane, polyethylene, tetrafluoroethylene, polytetrafluoroethylene, perfluoalkoxl, fluorinated ethylene propylene, or the like. The first tube member 30 may have a length, plus an outer diameter di, sufficient to extend through a working channel 91 of a conventional endoscope 90 (see FIGS. 6A-6B). The first tube member 30 also may comprise a hydrophilic coating overlying its outer surface. The hydrophilic coating, when applied to the outer surface of the first tube member 30, imparts suppleness and kink resistance to the tube member. The hydrophilic coating also may provide a lubricated surface to facilitate movement through the working channel 91 of the endoscope 90.

In the embodiment of FIGS. 1-6, the second tube member 40 has proximal and distal regions 42 and 44, respectively, and a lumen 45 extending therebetween. The second tube member 40 may comprise a relatively rigid material, such as stainless steel, compared to the first tube member 30. Advantageously, the provision of a relatively rigid second tube member 40 may reduce axial flexibility, relative to the first tube member, to facilitate capture of an object and/or reduce the likelihood of collapse along a distal segment of the retrieval device 20.

The second tube member 40 comprises an outer diameter d2, as best seen in FIGS. 4- 5, which is sized to be received at least partially within the lumen 35 of the first tube member 30. Thus, an inner diameter d, of the first tube member 30 (shown in FIG. 2A) is greater than the outer diameter d2 of the second tube member 40.

In the embodiment of FIGS. 1-6, the proximal region 42 of the second tube member 40 is disposed within the lumen 35 of the first tube member 30, while the distal region 44 of the second tube member 40 extends distally beyond the first tube member 30, as depicted in FIGS. 1-2. The second tube member 40 may comprise a relatively short overall length, as compared to an overall length of the first tube member 30. The proximal region 42 of the second tube member 40, along with a proximal region 62 of the arm 60, may be secured within the distal region 34 of the first tube member 30, as explained in further detail below.

The snare 50 comprises a loop member 55, which is coupled to a control member 51. A distal region 54 of the control member 51 is coupled to a proximal region 56 of the loop member 55, e.g., using an adhesive, solder, weld, mechanical coupling, or other suitable means.

As shown in FIGS. 1A-1B, a handle portion 70 may be used in conjunction with the retrieval device 20. In use, a proximal end 52 of the control member 51 is operably coupled to a slidable actuator 76, which may be moved relative to a thumb ring 75, as depicted in FIGS. IA- IB. In this embodiment, the handle portion 70 comprises a guide 77 having a slot 78 formed therein to permit longitudinal movement of the slidable actuator 76. The control member 51 extends proximally from within the lumen 35 of the first tube member 30, into the slot 78, and then is coupled to the slidable actuator 76, as depicted in FIGS. IA- IB. In use, longitudinal movement may be imparted to the control member 51 via the slidable actuator 76, which in turn is imparted to the loop member 55, as explained further below.

The arm 60 has proximal and distal regions 62 and 64. The proximal region 62 may be secured between the first and second tube members 30 and 40, respectively, as depicted in FIGS. 2A-2B. For example, a friction fit may be employed so that the proximal region 62 of the arm 60 is stabilized between the first and second tube members 30 and 40. In addition to, or in lieu of, a friction fit, the arm 60 may be secured between the first and second tube members 30 and 40 using an adhesive, solder or weld, tie-down bands, heat-shrink tubing, mechanical coupling, or the like.

The distal region 64 of the arm 60 may extend distally beyond the distal region 34 of the second tube member 40, as shown in FIGS. 1-2, and is coupled to a distal region 57 of the loop member 55. The distal region 64 of the arm 60 may extend substantially adjacent to an outer surface of the second tube member 40 in a collapsed state, as shown in FIG. 2A, but is not secured to the second tube member 40. Rather, the distal region 64 of the arm 60 may comprise a coupling mechanism, such as a hook or ring 65 having a groove or bore formed therein, for receiving the distal region 57 of the loop member 55, thereby coupling the arm 60 to the loop member 55. In this embodiment, the loop member 55 therefore may move with respect to the hook or ring 65. However, in alternative embodiments, the loop member 55 may be affixed relative to the arm 60, e.g., using an adhesive or solder.

Notably, the provision of the arm 60 does not increase the overall profile of the retrieval device 20. In particular, in the collapsed state shown in FIGS. IA and 2A, the arm 60 does not extend radially outward beyond the perimeter of first tube member 30. Advantageously, with a collapsed profile in which the arm 60 does not extend radially beyond the first tube member 30, the retrieval device 20 may be more easily advanced through a delivery device such as the endoscope 90.

In one presently preferred embodiment, the arm 60 is made from a shape-memory alloy such as nitinol and is configured to be biased to the deployed configuration shown in FIGS. IB, 2B and 3. More specifically, a shape memory material may undergo a substantially reversible phase transformation that allows it to "remember" and return to a previous shape or configuration. For example, in the case of nickel-titanium alloys, a transformation between an austenitic phase and a martensitic phase may occur by cooling and/or heating (shape memory effect) or by isothermally applying and/or removing stress (superelastic effect). Austenite is characteristically the stronger phase and martensite is the more easily deformable phase.

In an example of the shape memory effect, a nickel-titanium alloy having an initial configuration in the austenitic phase may be cooled below a transformation temperature (Mf) to the martensitic phase and then deformed to a second configuration. Upon heating to another transformation temperature (Af), the material may spontaneously return to its initial configuration. Generally, the memory effect is one-way, which means that the spontaneous change from one configuration to another occurs only upon heating. However, it is possible to obtain a two-way shape memory effect, in which a shape memory material spontaneously changes shape upon cooling as well as upon heating.

Applying these shape-memory properties to the arm 60, it will be possible to restrain the arm 60 radially inward during delivery by proximal positioning of the snare 50, which will pull the distal region 64 of the arm 60 towards the second tube member 40 as shown in FIGS. IA and 2A. When it is desired to deploy the loop member 55, the control member 51 is advanced distally, and the arm 60 may be biased to assume the configuration shown in FIGS. IB and 2B. The biased shape of the arm 60 may advantageously promote a consistent expanded configuration of the loop member 55.

Alternatively, the arm 60 may be made from other metals and alloys that may be biased to the configuration of FIGS. IB and 2B when no longer restrained. Solely by way of example, the arm 60 may comprise other materials such as stainless steel, cobalt-chrome alloys, amorphous metals, tantalum, platinum, gold and titanium. The arm 60 also may be made from non-metallic materials, such as thermoplastics and other polymers.

The arm 60 may comprise a cross-sectional shape that is rectangular, as shown in FIG. 4, or may comprise a concave curvature relative to the second tube member 40, as shown in FIG. 5. Alternatively, the arm 60 may comprise a cross-sectional shape that is round, square, triangular, pie-shaped, truncated cone, and the like, but preferably performs each of the functions outlined herein.

In the embodiment of FIG. 4, in which the arm comprises a rectangular cross- sectional profile, the arm 60 comprises a width w that may be about 0.2 to about 0.95 times the outer diameter d2 of the second tube member 40. Similarly, in the embodiment of FIG. 5, an estimated width w' of the arm 60' may be about 0.2 to about 0.95 times the outer diameter d2 of the second tube member 40. In the embodiments described below, particularly FIGS. 7-9, in which the second tube member 40 may be omitted, the widths w or w' may be about 0.2 to about 0.95 times the outer diameter dj of the first tube member 30.

Advantageously, the widths w or w' are sized to be considerably wider than a threadlike element. By providing a relatively wide rectangular or concave arm 60 or 60', several important clinical advantages may be achieved. For example, significantly enhanced grasping and pinching may be achieved when securing objects with the arm 60. Further, a relatively wide and strong arm 60 or 60' may have an enhanced column strength that allows the retrieval device 20 to be driven and/or pushed beyond objects in a passage when in the expanded state, and may reduce the risk of collapse of the arm 60 and snare 50 in the expanded state.

In operation, when the retrieval device 20 is used in conjunction with the endoscope 90, the endoscope may be maneuvered to a desired body passage 88. The endoscope 90 may be any conventional endoscope known in the art. For example, the endoscope 90 may comprise optical elements 93 and 94, which employ fiber optic components for illuminating and capturing an image distal to the endoscope. Optionally, in addition to the working lumen 91 noted above, the endoscope 90 may comprise an auxiliary lumen 92, as shown in FIGS. 6A-6B.

The endoscope 90 is maneuvered into the body passage 88 under visualization by the optical elements 93 and 94 with the retrieval device 20 in the collapsed state shown in FIGS. IA and 2 A. In this configuration, the slidable actuator 76 is positioned proximally within the slot 78, and therefore the control member 51 also is positioned proximally. A tension may be maintained upon the distal region 64 of the arm 60 to hold the arm 60 adjacent to the outer tube member 40, as best seen in FIG. 2A. In this state, the retrieval device 20 is delivered through the working lumen 91 of the endoscope 90.

When the endoscope 90 is at a desired location, e.g., positioned proximal to an object 99, the retrieval device 20 may be advanced such that the second tube member 40 is situated distal to the endoscope 90, as shown in FIG. 6A. Then, the slidable actuator 76 is advanced distally, as shown in FIG. IB, thereby urging the control member 51 distally and causing the loop member 55 to extend distal to the second tube member 40. At this time, tensile forces may be reduced or eliminated to allow the distal region 64 of the arm 60 to move in a radially outward direction relative to the first and second tube members 30 and 40, as shown in FIGS. IB, 2B, and 6A. In the expanded state, the loop member 55 may form a substantially circular or elliptical configuration.

In a next step, the retrieval device 20 is maneuvered, with visualization by the endoscope 90, so that the object 99 is positioned within the loop member 55 and/or between the arm 60 and an outer surface of the second tube member 40. The slidable actuator 76 then may be advanced proximally, thereby urging the loop member 55 back toward and/or partially into the second tube member 40. At this time, the loop member 55 is reduced in size and grasps the object 99. Notably, the distal region 64 of the arm 60 is simultaneously moved in a direction towards the second tube member 40, thereby further grasping and capturing the object 99. Thus, the object 99 is engaged and secured from multiple directions, i.e., using a combination of the loop member 55, the arm 60, and the second tube member 40. As noted above, by providing a relatively wide rectangular or concave arm 60 or 60', significantly enhanced grasping and pinching may be achieved when securing the object 99.

Depending on the procedure being performed, the object 99 may be captured and removed from the body, or moved to another passage. For example, if the object 99 is a polyp and the passage 88 is the colon, the retrieval device 20 may remove the polyp from the body. Alternatively, if the object 99 is a stone and the passage 88 is the bile duct, the stone may be moved proximally into the duodenum via the retrieval device 20, and then subsequently, the retrieval device 20 may be moved to the expanded state shown in FIG. 6A to allow the stone to be released into the duodenum to pass naturally through the body.

Referring now to FIGS. 7-8, in an alternative embodiment, the second tube member 40 is omitted. The alternative retrieval device 120 comprises a first tube member 130 having a proximal region (similar to the proximal region 32 of FIG. IA), a central region 133, and distal region 134. The distal region 134 comprises a groove 136, which may be an axially- oriented, stepped-down segment formed into the distal region 134. The groove 136 is sized to receive the arm 160 in the collapsed state, such that the arm 160 is substantially flush with the central region 133 of the first tube member 130, as depicted in FIGS. 7-8. In this embodiment, a proximal region 162 of the arm 160 may be secured to the first tube member 130 at or near the junction of the central and distal regions 133 and 134. For example, an angled bore 137 may be formed in the first tube member 130, and the proximal region 162 of the arm 160 may be secured in the angled bore 137 using a friction fit, adhesive, solder, weld, mechanical coupling, or the like. The distal region 164 of the arm 160, along with the snare 50 and other components not specifically described in FIG. 7, preferably are substantially identical to their respective counterparts described in FIGS. 1-6. Moreover, operation of the retrieval device 120 is generally similar to operation of the retrieval device 20, as described above, with a main exception that the object 99 may be engaged and secured from multiple directions using a combination of the loop member 55, the arm 160, and the distal region 134 of the first tube member 130 due to the omission of the second tube member 40.

In an alternative embodiment to FIGS. 7-8, the second tube member 40 of FIGS. 1-6 may be provided as generally described above, and the groove 136 may be formed in the second tube member 40. In this alternative, the first tube member may be provided in accordance with the first tube member 30 of FIGS. 1-6, i.e., without a groove formed therein, and the arm 160 is substantially flush with an outer surface of the second tube member in the collapsed state. Thus, the groove 136 may be formed either in the first or second tube member, depending on whether the second tube member is used.

Referring now to FIG. 9, in an alternative embodiment, the second tube member may be omitted, and an arm 260 may be formed integrally with a first tube member 230 by providing two longitudinal slits 238 and 239 in the distal region 234 of the first tube member. The arm 260 comprises a proximal region 262 in the vicinity of the termination of the slits 238 and 239, and further comprises a distal region 264 near the end of the first tube member 230. The distal region 264 of the arm 260 may be substantially co-extensive with the remainder of the first tube member 230 in the collapsed state, or the distal region 264 may extend distally beyond the first tube member 230, as shown in FIG. 9. In the latter embodiment, the slits 238 and 239 may be formed in the distal end of the first tube member 230, and then a distal portion of the first tube member 230, not encompassing the arm 260, may be cut to leave an elongated distal region 264 of the arm 260, which may provide leverage and other benefits during use.

The distal region 264 of the arm 260 may be coupled to the loop member 55 of the snare 50 via the hook or ring 56, as described above. Moreover, operation of the retrieval device 220 is generally similar to operation of the retrieval devices 20 and 120, as described above.

In an alternative embodiment to FIG. 9, the second tube member 40 of FIGS. 1-6 may be provided as generally described above, and the slits 238 and 239 may be formed in the second tube member 40. In this alternative, the first tube member may be provided in accordance with the first tube member 30 of FIGS. 1-6, i.e., without slits formed therein, and the arm 260 is formed integrally with the second tube member 40. Thus, an integral arm 260 may be formed either in the first or second tube member, depending on whether the second tube member is used.

Referring now to FIG. 10, a further alternative retrieval device 320 is similar to the retrieval device of FIGS. 1-6, with a main exception that multiple arms 6OA and 6OB are provided and coupled to the loop member 55 at first and second locations 56A and 56B. The first and second arms 6OA and 6OB may be secured between the first and second tube members 30 and 40 at spaced apart circumferential locations. Optionally, the multiple arms 6OA and 6OB may be disposed within corresponding grooves formed in either the first or second tube member, as generally explained in FIGS. 7-8 above. Alternatively, the multiple arms 6OA and 6OB may be formed integrally with either the first or second tube member, as generally explained in FIG. 9 above. Operation of the retrieval device 320 therefore is generally similar to operation of the retrieval devices described above, depending on which particular configuration is used. Advantageously, if multiple arms 6OA and 6OB are provided, an increased surface area around the circumference of the device 20 may be provided to facilitate capture and removal of objects from the body. If desired, greater than two arms may be employed.

Referring now to FIGS. 1 IA-I IB, a further alternative retrieval device 420 is shown. The general assembly of the retrieval device 420 may be similar to the retrieval devices noted above, and may include a first tube member 430, a second tube member 440, and a plurality of arms 460a-460c. The plurality of arms 460a-460c may be similar to the arms described above. In this embodiment, first and second control members 451 and 452 are provided. The first control member 451 may extend through a lumen of the first and second tube members 430 and 440 and may be coupled to proximal regions of the plurality of arms 460a-460c, thereby permitting proximal and distal movement of the arms. The second control member 452 may extend within a tube 453 adjacent to the first tube member 430. The second control member 452 is coupled to a closing ring 458, which extends circumferentially through loop elements 463 that are coupled to distal ends of the plurality of arms 460a-460c, as shown in FIGS. 1 IA-I IB.

In use, the plurality of arms 460a-460c may be deployed and positioned to surround an object 99, as shown in FIG. 1 IA. In a next step, the second control member 452 may be retracted proximally to reduce the size of the closing ring 458, thereby effectively closing off a distal region of the plurality of arms 460a-460c. At this time, the object 99 is captured between the plurality of arms 460a-460c, as shown in FIG. 1 IB.

While various embodiments of the invention have been described, it will be apparent to those of ordinary skill in the art that many more embodiments and implementations are possible within the scope of the invention. Accordingly, the invention is not to be restricted except in light of the attached claims and their equivalents.

## Claims

1. Apparatus for retrieving an object from a body passage, the apparatus comprising: a first tube member having proximal and distal regions and lumen extending therebetween; a second tube member having proximal and distal regions and a lumen extending therebetween, wherein at least the distal region of the second tube member extends distal to the first tube member, and wherein at least one of the outer diameter or axial flexibility of the first and second tube members is different from one another; a snare comprising a loop member, wherein a proximal region of the loop member is coupled to a control member; and an arm having proximal and distal regions, wherein the proximal region of the arm is secured between the first and second tube members, and wherein the distal region of the arm is coupled to the loop member, wherein proximal and distal advancement of the control member moves the loop member between collapsed and expanded states, respectively.

2. The apparatus of claim 1 wherein the second tube member is more rigid than the first tube member.

3. The apparatus of claim 1 or 2 wherein the arm is configured to be biased into a predetermined configuration when the loop member is in the expanded state.

4. The apparatus of claim 1, 2 or 3 wherein the arm comprises a substantially rectangular cross-sectional profile.

5. The apparatus of the preceding claims wherein the arm comprises a concave curvature relative to the second tube member.

6. The apparatus of any of the preceding claims wherein the second tube member is disposed at least partially within the lumen of the first tube member, and wherein the proximal region of the arm is secured between the first and second tube members using a friction fit.

7. The apparatus of any of the preceding claims wherein the distal region of the first tube member comprises a groove that is sized to receive the arm in the collapsed state, such that the arm is substantially flush with the central region of the first tube member in the collapsed state.

8. The apparatus of any of the preceding claims wherein the arm comprises a width that is about 0.2 to about 0.95 times an outer diameter of the first tube member.

9. The apparatus of any of the preceding claims wherein the second tube member is disposed at least partially within the lumen of the first tube member, and wherein at least the distal region of the second tube member extends distal to the first tube member.

10. The apparatus of any of the preceding claims wherein the second tube member comprises a groove that is sized to receive the arm in the collapsed state, such that the arm is substantially flush with an outer surface of the second tube member in the collapsed state.

11. The apparatus of any of the preceding claims wherein first and second arms are coupled to the loop member of the snare at first and second locations, respectively.

12. Apparatus for retrieving an object from a body passage, the apparatus comprising: a first tube member having proximal and distal regions, a lumen extending therebetween, and a central region having a first outer diameter; a snare comprising a loop member, wherein a proximal region of the loop member is coupled to a control member; and an arm having proximal and distal regions, wherein the proximal region of the arm is secured to the first tube member and the distal region of the arm is coupled to the loop member, wherein proximal and distal advancement of the control member moves the loop member between collapsed and expanded states, respectively, and wherein the arm does not extend radially beyond the first outer diameter of the first tube member in the collapsed state.

13. The apparatus of claim 12 wherein the distal region of the first tube member comprises a groove that is sized to receive the arm in the collapsed state, such that the arm is substantially flush with the central region of the first tube member in the collapsed state.

14. The apparatus of claim 12 or 13 wherein the arm is formed integrally with the first tube member by providing two longitudinal slits in the distal end of the first tube member.

15. The apparatus of claim 12, 13 or 14 wherein the arm comprises a width that is about 0.2 to about 0.95 times an outer diameter of the first tube member.

16. The apparatus of any of claims 12-15 further comprising a second tube member having proximal and distal regions and a lumen extending therebetween, wherein the second tube member is disposed at least partially within the lumen of the first tube member, and wherein at least the distal region of the second tube member extends distal to the first tube member.

17. The apparatus of any of claims 12-16 wherein the arm is formed integrally with the second tube member by providing two longitudinal slits in the distal end of the second tube member.

18. The apparatus of any of claims 12-17 wherein the second tube member comprises a groove that is sized to receive the arm in the collapsed state, such that the arm is substantially flush with an outer surface of the second tube member in the collapsed state.

19. The apparatus of any of claims 12-18 wherein first and second arms are coupled to the loop member of the snare at first and second locations, respectively.
